Europäisches Patentamt

European Patent Office

Office européen des brevets

(19)

(11) Numéro de publication : **0 178 207**
**B1**

(12)

# FASCICULE DE BREVET EUROPÉEN

(45) Date de publication du fascicule du brevet :
02.03.88

(21) Numéro de dépôt : 85401788.6

(22) Date de dépôt : 16.09.85

(51) Int. Cl.⁴ : **F 25 J   3/08**, F 25 J   3/02,
C 07 C   9/02, C 07 C   7/09

(54) **Procédé et installation de fractionnement cryogénique de charges gazeuses.**

(30) Priorité : 28.09.84 FR 8414996

(43) Date de publication de la demande :
16.04.86 Bulletin 86/16

(45) Mention de la délivrance du brevet :
02.03.88 Bulletin 88/09

(84) Etats contractants désignés :
BE DE GB IT NL SE

(56) Documents cités :
AT-B-   449 816
US-A- 4 251 249
US-A- 4 322 225

(73) Titulaire : **COMPAGNIE FRANCAISE D'ETUDES ET DE CONSTRUCTION "TECHNIP"**
**170 Place Henri Regnault**
**F-92090 Paris la Défense (FR)**

(72) Inventeur : **Paradowski, Henri**
**32 rue Serpente**
**F-95000 Cergy-Pontoise (FR)**
Inventeur : **Castel, Joelle**
**61 rue du Val André**
**F-78560 Le Pont Marly (FR)**
Inventeur : **Parfait, Hervé**
**53, rue Chavlot**
**F-75003 Paris (FR)**

(74) Mandataire : **Durand, Yves Armand Louis et al**
**Cabinet Z. Weinstein 20, Avenue de Friedland**
**F-75008 Paris (FR)**

Jouve, 18, rue St-Denis, 75001 Paris, France

## Description

La présente invention a essentiellement pour objet un procédé de fractionnement cryogénique de charges gazeuses quelconques et telles que par exemple les gaz naturels, les gaz associés à des condensats d'hydrocarbure, ou les gaz issus du traitement de fractions pétrolières.

Elle vise également une installation comportant application de ce procédé.

On a déjà proposé de nombreuses installations industrielles permettant le fractionnement de charges gazeuses en un gaz résiduaire contenant les composés les plus volatils de la charge et en un produit liquide contenant les composés les plus lourds de la charge, cela en vue d'obtenir dans ledit produit liquide un composant donné de la charge avec un taux de récupération élevé.

A cet égard, on peut citer par exemple la récupération du gaz de pétrole liquéfié (hydrocarbures en $C_3$-$C_4$) à partir de gaz naturel ou de raffinerie, la récupération d'éthane destinée en particulier à alimenter des unités de vapo-craquage, ou la désulfuration et le dégazolinage de gaz naturels par récupération des composés soufrés tels que l'oxysulfure de carbone et les mercaptans.

D'une manière générale, dans toutes ces installations connues, la charge gazeuse est partiellement condensée par refroidissement à basse température puis séparée dans un ballon séparateur. Puis, la partie liquide est traitée dans une colonne de fractionnement classique, et on récupère en fond de cette colonne sous la forme liquide le ou les composés lourds de la charge que l'on souhaite obtenir. Dans certains cas, on prévoit un cycle frigorifique pour assurer les besoins en froid de l'installation.

Toutefois, ce genre d'installations présente des inconvénients, car si l'on veut obtenir en fond de colonne de fractionnement tel ou tel composé lourd avec un taux de récupération élevé, il faut condenser une grande partie des composés légers en amont de la colonne de fractionnement, ce qui, comme on le comprend, nécessite une basse température lors de la réfrigération de la charge gazeuse ainsi que dans le système de tête de la colonne de fractionnement.

En d'autres termes, dans les installations connues utilisant un simple ballon séparateur, il est nécessaire de condenser beaucoup de composés légers pour condenser tous les composés lourds, ce qui implique une fourniture de frigories importante de sorte que ces installations consomment beaucoup d'énergie en raison de la puissance requise pour produire le froid.

On retrouve ce même problème dans le document US-A-4322. 225 qui décrit un procédé et une installation de traitement du gaz naturel utilisant notamment une tour de séparation, associée à une colonne de fractionnement pour séparer les composés légers des composés lourds.

L'invention a donc principalement pour but de résoudre ce problème majeur, en proposant un procédé et une installation qui permettent de réduire au minimum la quantité de composés légers introduits dans la colonne de fractionnement, de façon à diminuer notablement la consommation d'énergie de l'installation et tout particulièrement la puissance consommée pour fournir le froid.

A cet effet, l'invention a pour objet un procédé de fractionnement cryogénique de charges gazeuses quelconques en un gaz résiduaire contenant les composés les plus volatils de la charge en un produit liquide contenant les composés les plus lourds de cette charge, ce procédé consistant à injecter ladite charge partiellement condensée en fond d'une colonne de purification-réfrigération par contact, laquelle colonne produit en tête tout ou partie du gaz résiduaire et en fond un liquide que l'on injecte dans une colonne de fractionnement pour obtenir en fond de cette colonne un produit liquide contenant les composés les plus lourds de la charge, et étant caractérisé en ce que le distillat en tête de la colonne de fractionnement est au moins partiellement condensé et injecté comme alimentation de tête de ladite colonne de purification-réfrigération pour ainsi récupérer dans le liquide au fond de cette colonne les composés lourds présents dans la fraction vaporisée de la charge gazeuse.

On comprend donc déjà que la prévision d'une colonne de purification-réfrigération par contact à la place d'un simple ballon séparateur sur la charge gazeuse partiellement condensée permettra avantageusement la récupération du composé désiré en fond de cette colonne grâce à l'alimentation essentiellement liquide injectée en tête et qui assure le refroidissement et la condensation sélective des composés lourds de la fraction gazeuse avec un minimum de condensation de composés plus légers. Dès lors, les températures de refroidissement de la charge et en tête de la colonne de fractionnement seront plus élevées de sorte que la consommation d'énergie pour produire le froid sera nettement réduite.

Selon une autre caractéristique du procédé de l'invention, l'alimentation de tête de la colonne de purification-réfrigération précitée est constituée par le distillat vapeur de la colonne de fractionnement, partiellement ou totalement condensé dans un système d'échange et/ou par le distillat liquide de ladite colonne de fractionnement, sous-refroidi ou non dans ledit système.

Ce procédé est encore caractérisé par le fait que la colonne de fractionnement précitée fonctionne à une pression supérieure à celle de la colonne de purification-réfrigération par contact pour permettre l'injection du distillat de la colonne de fractionnement dans la colonne de purification-réfrigération sans compression dudit distillat.

Suivant une autre caractéristique du procédé, un appoint de frigories à bas niveau de température, nécessaire au refroidissement à basse température de la charge gazeuse et à sa condensation partielle avant injection dans la colonne de purification-réfrigération, nécessaire au refroidissement du ou des distillats de la colonne de fractionnement, et également nécessaire au système de condensation de cette

2

colonne, est fourni par un système de réfrigération.

Suivant un mode de réalisation, le système de réfrigération est constitué par un cycle frigorifique à compression dans lequel un fluide frigorifique comprenant un ou plusieurs corps purs est porté à haute pression dans un compresseur, puis condensé et éventuellement sous-refroidi à cette pression et enfin détendu à la ou les pressions compatibles avec les niveaux de température des systèmes d'échange du procédé pour être vaporisé dans lesdits systèmes.

Selon un autre mode de réalisation, le système de réfrigération précité est constitué par la détente de la charge gazeuse dans un turbo-expandeur assurant la condensation partielle de ladite charge et produisant un travail mécanique utilisé pour entraîner une machine tournante, tandis qu'un complément de frigories est éventuellement fourni par le cycle frigorifique précité.

On peut bien entendu utiliser toute autre installation de détente permettant la condensation partielle de la charge gazeuse et produisant ou non un travail extérieur, cela sans sortir du cadre de l'invention.

L'invention vise encore une installation pour la mise en œuvre du procédé ci-dessus, cette installation comprenant une colonne de purification-réfrigération par contact associée à une colonne de fractionnement et étant caractérisée par au moins une conduite d'alimentation de distillat reliant la tête de la colonne de fractionnement à la tête de la colonne de purification-réfrigération après passage dans un système d'échange en amont de cette dernière, ainsi que par un conduit reliant le fond de la colonne de purification-réfrigération à la colonne de fractionnement après passage dans ledit système d'échange.

On précisera ici qu'au moins une conduite d'arrivée de la charge gazeuse est connectée en fond de la colonne de purification-réfrigération et qu'une conduite d'évacuation du gaz résiduaire est reliée à la tête de ladite colonne, ces deux conduites traversant le système d'échange précité.

Suivant une autre caractéristique de cette installation, la tête de la colonne de fractionnement est munie d'un condenseur et d'un ballon de reflux comportant d'une part un premier conduit d'alimentation en distillat gazeux passant dans le système d'échange avant d'aboutir à la tête de la colonne de purification-réfrigération, et d'autre part un deuxième conduit de reflux du distillat liquide dans la tête de la colonne de fractionnement, ce deuxième conduit étant éventuellement relié à la tête de la colonne de purification-réfrigération par une conduite traversant ledit système d'échange pour alimenter dans ladite colonne un distillat liquide sous-refroidi.

Cette installation est encore caractérisée en ce qu'au système d'échange précité est éventuellement associé un circuit de fluide réfrigérant.

Suivant un autre mode de réalisation, la tête de la colonne de fractionnement est équipée d'un condenseur auquel du froid est fourni par un circuit de fluide réfrigérant associé au système d'échange précité.

Selon encore un autre mode de réalisation de cette installation, la tête de la colonne de fractionnement comporte une conduite de distillat vapeur traversant le système d'échange précité et aboutissant à un ballon de reflux du distillat liquide dans ladite colonne, lequel ballon comporte une conduite d'alimentation en distillat gazeux passant dans ledit système d'échange avant d'aboutir à la tête de la colonne de purification-réfrigération.

On précisera ici que la conduite d'arrivée de la charge gazeuse, après passage dans le système d'échange, est reliée à un séparateur dont le fond comporte une voie d'écoulement de la fraction liquide débouchant dans le fond de la colonne de purification-réfrigération et dont la tête comporte une voie de passage de la fraction gazeuse aboutissant à l'entrée d'un turbo-expandeur dont la sortie est raccordée au fond de ladite colonne.

Suivant encore une autre caractéristique de la variante d'installation ci-dessus, le turbo-expandeur précité entraîne au moins une machine tournante, telle que par exemple un compresseur relié à la conduite d'évacuation du gaz résiduaire.

Mais d'autres caractéristiques et avantages de l'invention apparaîtront mieux dans la description détaillée qui suit et se réfère aux dessins annexés, donnés uniquement à titre d'exemple, et dans lesquels :

La figure 1 est une vue schématique d'une installation de fractionnement de charges gazeuses, conforme aux principes de l'invention ;

La figure 2 illustre schématiquement un exemple de réalisation d'une installation conforme à l'invention et utilisant un système à réfrigération directe par un cycle frigorifique, pour récupérer les composés d'hydrocarbure C3 + à partir d'un gaz de raffinerie, et

La figure 3 illustre schématiquement un autre exemple de réalisation d'une installation conforme à l'invention et permettant le dégazolinage des gaz naturels.

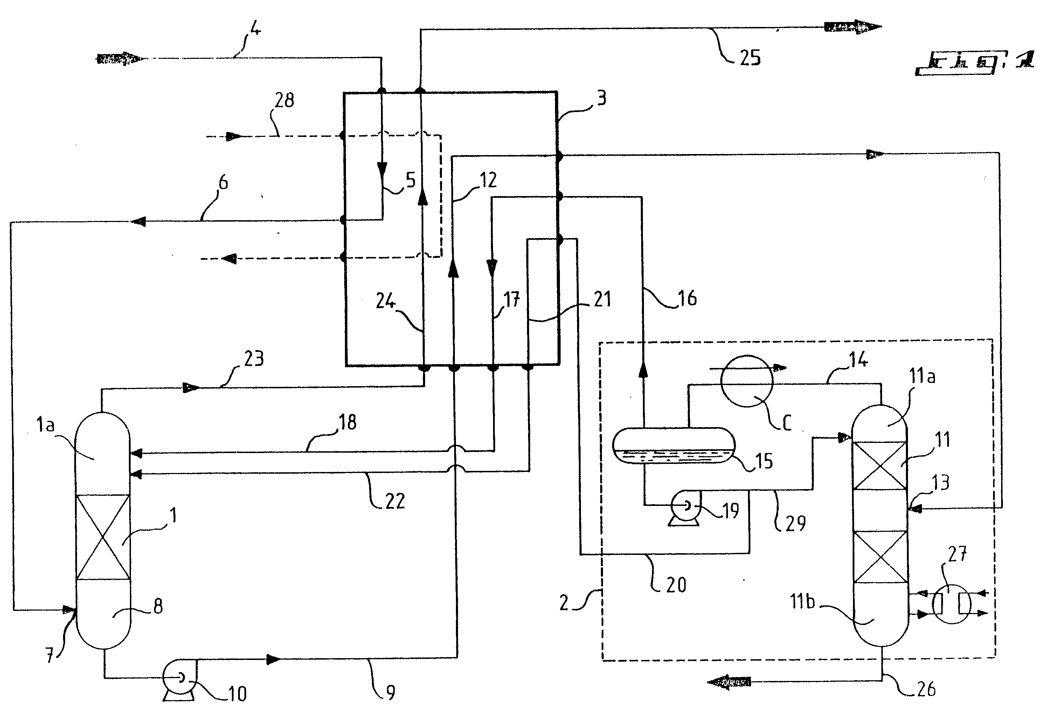

On se reportera tout d'abord à la figure 1 qui illustre le principe d'une installation selon l'invention et dont on décrira ci-après la structure et le fonctionnement.

Cette installation comprend essentiellement une colonne 1 de purification-réfrigération par contact, un ensemble 2 formant colonne de fractionnement et un système d'échange 3.

La charge gazeuse à traiter et qui se compose d'un mélange de composés lourds et légers parvient à l'installation par une conduite 4 après avoir subi une phase de compression qui n'est pas représentée. A la conduite 4 fait suite une portion de conduite 5 passant dans le système d'échange 3 où ladite phase gazeuse est refroidie pour donner un fluide partiellement condensé qui est injecté en 7, par la portion de conduite 6, dans le fond de la colonne 1 de purification-réfrigération. Le liquide 8 en fond de cette colonne et contenant des composés lourds avec un minimum de composés légers est envoyé par une conduite 9 équipée d'une pompe 10 à une colonne de fractionnement 11 faisant partie de l'ensemble de fractionnement 2. Plus précisément, à la conduite 9 fait suite une portion de conduite 12 dans laquelle la partie liquide 8 est réchauffée avant d'être injectée en 13 dans la colonne de fractionnement 11.

Cette colonne 11 est munie en tête d'un condenseur à réfrigérant externe C placé sur un conduit 14 reliant la tête 11a de la colonne 11 à un ballon de reflux 15. Le condenseur C assure la condensation au moins partielle de la fraction gazeuse sortant en tête de la colonne 11 et contenant un minimum de composés légers.

Le ballon de reflux 15 comporte un premier conduit 16 d'alimentation en distillat gazeux qui est refroidi dans le système d'échange 3 en passant dans la portion de conduite 17 pour être condensé au moins partiellement et ainsi injecté par la portion de conduit 18 en tête 1 de la colonne de purification-réfrigération.

Le ballon de reflux 15 comporte un deuxième conduit 29 équipé d'une pompe 19 et assurant le reflux en tête 11a de la colonne 11.

Suivant un mode de réalisation préféré, le distillat liquide peut être injecté en tête 1a de la colonne 1 au moyen d'une conduite 20 reliant la conduite de reflux 29 à ladite tête 1a, comme on le voit bien sur la figure. Plus précisément, la conduite 20 comporte une portion 21 passant dans le système d'échange 3 où le distillat liquide est sous-refroidi pour parvenir par le conduit 22 à la tête 1a de la colonne de purification-réfrigération 1.

En tête 1a de cette colonne sort le gaz résiduaire par une conduite 23, lequel gaz résiduaire est réchauffé dans le système d'échange 3 en passant par la portion de conduite 24 avant de sortir de l'installation par la conduite 25.

Bien entendu, on recueille en fond 11b de la colonne de fractionnement 11 un produit liquide comprenant le ou les composés lourds qui sortent par une conduite 26. Un rebouilleur 27 est associé au fond 11b de la colonne de fractionnement 11.

Enfin, on a montré schématiquement en 28 un circuit de fluide réfrigérant associé au système d'échange 3 et qui peut être éventuellement prévu pour fournir l'appoint de froid nécessaire à l'installation.

Le fonctionnement de l'installation se déduit immédiatement de la description qui précède, mais on insistera ici sur le rôle de la colonne 1 de purification-réfrigération qui est essentiel.

En effet, grâce aux alimentations totalement ou partiellement liquides 18 et 22 injectées en tête de la colonne 1, les composés lourds de la fraction vaporisée dans cette colonne seront condensés et récupérés en fond de celle-ci avec un minimum de condensation de composés plus légers, le gaz résiduaire 23 sortant en tête 1a de ladite colonne étant très froid et plus froid que les alimentations liquides 18 et 22.

Il en résulte que par rapport aux installations classiques utilisant un simple ballon séparateur, l'installation selon l'invention procure de nombreux avantages en ce que notamment les températures de refroidissement de la charge gazeuse et dans le condenseur de tête de la colonne de fractionnement peuvent être élevées, et en ce que on diminue la charge de la colonne de fractionnement et la charge thermique de son condenseur de tête.

Dans ces conditions, la quantité d'énergie consommée par une telle installation est notablement réduite ainsi que le coût des équipements d'échange et de compression.

Mais les avantages de l'invention seront encore illustrés par la description qui va suivre de deux modes de réalisation donnés à titre d'exemples non limitatifs des nombreuses applications que peut recevoir cette invention.

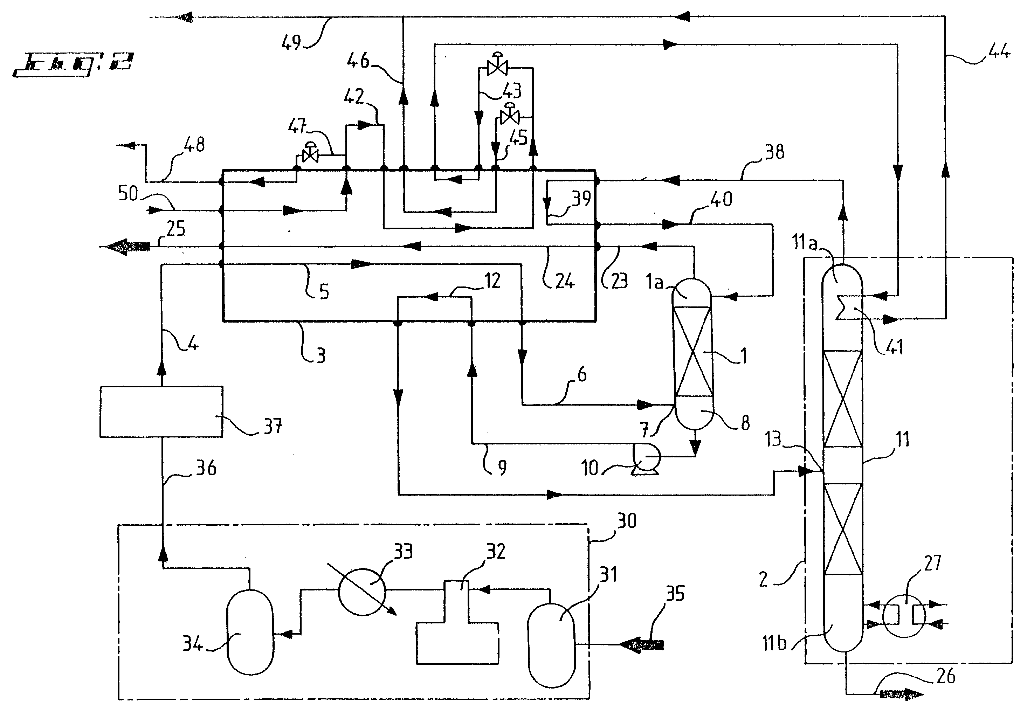

On se reportera tout d'abord à la figure 2 qui illustre une installation mettant en œuvre les principes de l'invention en association avec un système à réfrigération directe par un cycle frigorifique pour récupérer la coupe C3 + à partir d'une charge gazeuse constituée par un gaz de raffinerie à basse pression (5 bar) dont la composition est la suivante :

| Composants | : | % en volume |
|------------|---|-------------|
| Hydrogène | : | 12 |
| Azote | : | 4 |
| CO2 | : | 2 |
| Méthane | : | 37 |
| Coupe C2 | : | 23 |
| Coupe C3 | : | 18 |
| Coupe C4 + | : | 4 |

On notera que le taux de récupération de la coupe C3 + désiré est de 99 %.

Sur la figure 2, on a utilisé les mêmes repères que ceux de la figure 1 pour désigner les éléments communs, et à savoir les éléments essentiels d'une installation conforme aux principes de l'invention.

4

On voit sur la figure 2 que la charge gazeuse à traiter passe tout d'abord dans une section de compression 30 comprenant un ballon d'aspiration 31, un compresseur 32, un refroidisseur 33 du gaz, et un ballon séparateur 34. Cette section de compression 30 comprime le gaz de charge 35 jusqu'à 6,2 bar, et le gaz ainsi comprimé parvient par un conduit 36 à une section de séchage 37 qui comme connu en soi, produit du gaz sec qui, comme cela a été décrit à propos de la figure 1, est injecté en 7 au fond de la colonne de purification-réfrigération 1 après passage dans la conduite 4, dans la conduite 5 passant dans le système d'échange 3 et dans la conduite 6. Le gaz est ainsi refroidi jusqu'à une température d'environ — 62 °C, et le fluide sortant du système d'échange 3 par la conduite 6 reliée à la colonne 1 se trouve à une pression de 5,1 bar.

On voit en 38 une conduite dans laquelle passe le distillat sortant de la colonne de fractionnement 11 et qui est presque totalement condensé après passage 39 dans le système d'échange 3 pour être finalement injecté par le conduit 40 en tête 1a de la colonne de purification-réfrigération. Le fluide dans la conduite 40 est à une température d'environ — 62 °C.

Le gaz sortant en 23 de la colonne 1, à 5 bar, est réchauffé jusqu'à 29 °C dans le système d'échange 3 pour donner, comme décrit à propos de la figure 1, un gaz résiduaire envoyé à l'extérieur de l'installation par la conduite 25.

Le liquide 8 issu du fond de la colonne 1 est pompé à 6,6 bar dans la pompe 10, puis est réchauffé à — 25 °C dans le système d'échange 3 (conduite 12), pour donner un flux injecté en 13 dans la colonne de fractionnement 11 qui est équipée en fond d'un rebouilleur 27 et en tête 11a d'un condenseur intégré 41. Cette colonne de fractionnement 11 fonctionne à une pression de 6,3 bar et produit en tête 11a le distillat vapeur précité et sortant par la conduite 38, et en fond la coupe C3 + liquide qui est envoyée à l'extérieur de l'installation par la conduite 26.

Le froid au condenseur 41 est fourni par un circuit de fluide réfrigérant associé au système d'échange 3 et qui sera décrit ci-après.

Le froid est fourni au condenseur 41 par une partie du fluide réfrigérant 42 détendu à basse pression en 43 et vaporisé en 44 après passage dans 41. Le reste du fluide réfrigérant liquide 42 est détendu à basse pression en 45 puis vaporisé dans le système d'échange 3 en 46 de façon à assurer les besoins en froid à basse température dudit système d'échange 3.

Une partie 47 du fluide réfrigérant liquide est vaporisée à moyenne pression dans le système d'échange pour donner le flux 48, ce qui permet d'apporter le froid à moyenne température nécessaire pour le procédé.

Le fluide réfrigérant vapeur 49 à basse pression, qui est un mélange des fluides 44 et 46 est retourné à l'étage basse pression du compresseur (non représenté) du cycle frigorifique.

Le fluide réfrigérant vapeur 48 à moyenne pression est retourné à l'étage moyenne pression du compresseur précité.

Enfin, au refoulement de ce compresseur, le fluide réfrigérant est condensé dans un échangeur classique et non représenté, puis renvoyé à l'installation pour être réutilisé en 50.

La demanderesse a constaté que, dans l'installation ci-dessus, on réduisait la puissance consommée par le cycle frigorifique de 20 % par rapport aux installations classiques, c'est-à-dire par rapport aux installations qui n'utilisent pas une colonne de purification-réfrigération par contact, mais un simple ballon séparateur.

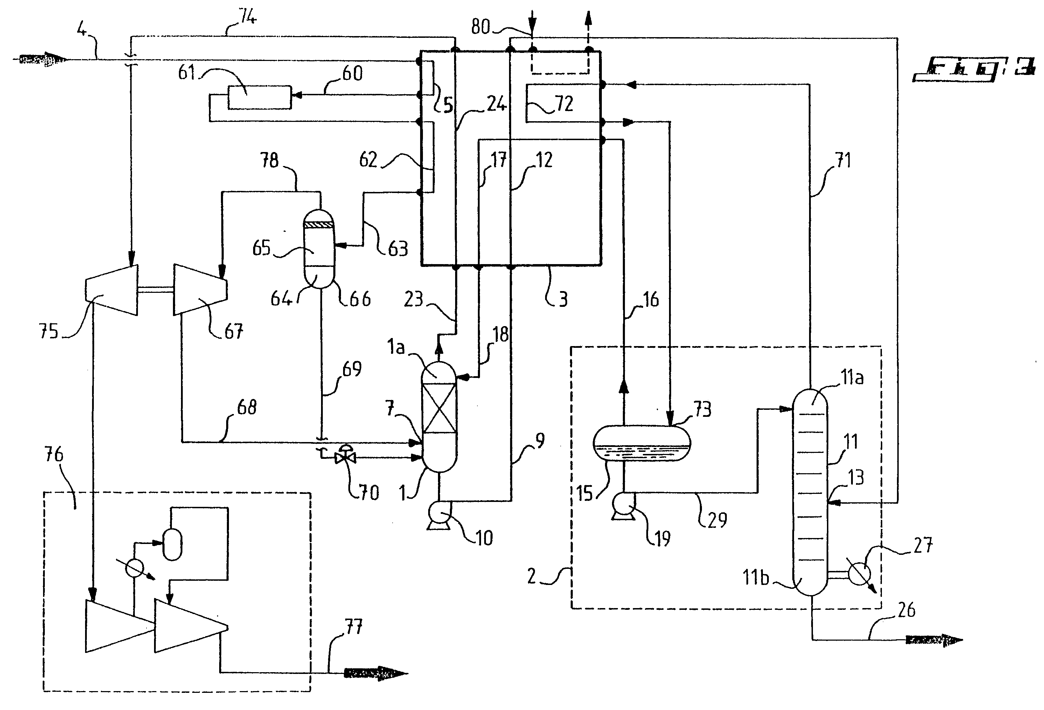

On se reportera maintenant à la figure 3 qui représente une unité de dégazolinage de gaz naturel dont la composition est la suivante :

| Composants | % molaire |
|---|---|
| Azote | 0,5 |
| CO2 | 3,1 |
| Méthane | 65,5 |
| Ethane | 13,4 |
| Propane | 9,4 |
| Butanes | 5,1 |
| Pentanes | 2 |
| Hexane et plus lourds | 1 |

Cette unité permet la récupération des GPL (coupe C3/C4) avec un taux de récupération des C4 qui est supérieur à 95 % et avec un rapport C3/C4 dans cette coupe, égal à 30/70 en volume.

On a utilisé sur la figure 3 les mêmes repères que sur la figure 1 pour désigner les éléments communs.

La charge gazeuse qui est disponible à basse pression (5 bar) est comprimée dans une section de compression (non représentée) jusqu'à 49 bar pour arriver dans l'installation par la conduite 4.

Le flux gazeux passe ensuite dans la conduite 5 et est ainsi refroidi dans le système d'échange 3 jusqu'à 20 °C. Puis il parvient par une conduite 60 dans une section de séchage 61 connue en soi.

Le gaz séché est alors refroidi en 62 jusqu'à environ 13 °C par passage dans le système d'échange 3 pour donner un flux 63 qui, à ce niveau, est partiellement condensé.

Les phases liquide 64 et vapeur 65 sont séparées dans un ballon 66.

La phase vapeur 65 parvient par une conduite 78 à l'entrée d'un turbo-expandeur 67, de sorte qu'à la sortie de ce turbo-expandeur, ladite phase vapeur se trouve détendue à une pression de 16 bar environ, et le flux 68 alimente en 7 le fond de la colonne 1 de purification-réfrigération.

La phase liquide 64 du ballon 66 alimente aussi le fond de la colonne 1 par passage dans une conduite 69 équipée d'une vanne de détente 70.

Le distillat vapeur sortant de l'ensemble 2 formant colonne de fractionnement passe dans la conduite 16, est condensé en 17 dans le système d'échange 3 et est injecté par la conduite 18 en tête 1a de la colonne 1 comme on l'a décrit à propos de la figure 1. Il convient de préciser que ce distillat vapeur est condensé dans le système d'échange 3 par le gaz résiduaire froid 23 sortant de la colonne 1 et par le flux liquide sortant du fond de ladite colonne 1 par la conduite 9.

Ce flux est réchauffé jusqu'à environ 40 °C puis est injecté en 13 dans la colonne de fractionnement 11 qui produit en fond 11b la coupe C3 + recueillie en 26, et en tête un distillat vapeur qui sort par une conduite 71. Ce distillat est refroidi et condensé en 72 dans le système d'échange 3, puis injecté en 73 dans le ballon de reflux 15 déjà décrit à propos de la figure 1. On retrouve donc ici la conduite 29 de reflux du distillat liquide et la conduite 16 d'alimentation du distillat vers la colonne 1 de purification-réfrigération après condensation au moins partielle dans le système d'échange 3.

Les frigories nécessaires au système de condensation de la colonne de fractionnement 11 sont apportées dans la section d'échange 3, par un circuit 80 de fluide réfrigérant tel que par exemple du propane.

Le gaz résiduaire produit en 23 et sortant de la colonne 1 est réchauffé jusqu'à 40 °C environ dans le système d'échange 3, puis parvient par la portion de conduite 74 à un compresseur 75 attelé au turbo-expandeur 67. Enfin, le gaz comprimé en 75 est envoyé dans une section de compression 76 qui n'a pas besoin d'être décrite en détail ici, et qui permet de délivrer le gaz résiduaire en 77 à une pression élevée d'environ 82 bar.

On a constaté que, par rapport à une installation classique comportant un simple ballon séparateur, la mise en œuvre de l'invention dans l'installation telle que représentée sur la figure 3 présente de nombreux avantages.

Elle permet notamment d'améliorer le taux d'extraction de butane, de 95 % dans l'installation avec ballon-séparateur, à 99 % dans l'installation de la figure 3, et cela sans modifier les compresseurs de charge gazeuse et de gaz produit.

Elle permet en outre une simplification notable du cycle frigorifique au propane qui ne comporte qu'un seul étage de compression (alors que dans l'installation classique il était nécessaire d'en utiliser deux), et cela grâce à un meilleur ajustement des niveaux de températures et à la réduction de la charge thermique du condenseur et du refroidissement de la charge. Ceci représente une économie de puissance de l'ordre de 10 %, ainsi qu'une réduction du coût des équipements.

Ces avantages apparaissent dans le tableau ci-après :

| | Schéma classique | Schéma selon l'invention |
|---|---|---|
| Taux d'extraction de butane | 95% | 99% |
| Energie consommée | | |
| Compresseur de charge | 11 MW | 11 MW |
| Compresseur de gaz produit | 6,6 MW | 6,6 MW |
| Cycle frigorifique | 1,6 MW | 0,2 MW |
| TOTAL | 19,2 MW | 17,8 MW |

Grâce à la présente invention, on peut donc réaliser des installations qui, pour un même débit de charge gazeuse et pour un même taux de récupération d'un ou plusieurs composés de la charge dans le liquide produit en fond de la colonne de fractionnement, permettent de réduire la consommation d'énergie de 10 à 40 % par rapport aux installations classiques mettant en œuvre la même technique de réfrigération, mais ne mettant pas en œuvre une colonne de purification-réfrigération par contact associée à une colonne de fractionnement.

**Revendications**

1. Procédé de fractionnement cryogénique de charges gazeuses quelconques en un gaz résiduaire contenant les composés les plus volatils de la charge et en un produit liquide contenant les composés les plus lourds de cette charge, ce procédé consistant à injecter ladite charge partiellement condensée en fond d'une colonne de purification-réfrigération par contact, laquelle colonne produit en tête tout ou partie dudit gaz résiduaire et en fond un liquide que l'on injecte dans une colonne de fractionnement pour obtenir en fond de cette colonne un produit liquide contenant les composés les plus lourds de la charge, caractérisé en ce que le distillat en tête de la colonne de fractionnement est au moins partiellement condensé et injecté comme alimentation de tête de la colonne de purification-réfrigération pour ainsi récupérer dans le liquide au fond de cette colonne les composés lourds présents dans la fraction vaporisée de la charge gazeuse.

2. Procédé selon la revendication 1, caractérisé en ce que l'alimentation de tête de la colonne de purification-réfrigération précitée est constituée par le distillat vapeur de la colonne de fractionnement, partiellement ou totalement condensé dans un système d'échange, et/ou par le distillat liquide de ladite colonne de fractionnement, sous-refroidi ou non dans ledit système d'échange.

3. Procédé selon la revendication 1 ou 2, caractérisé en ce que la colonne de fractionnement précitée fonctionne à une pression supérieure à celle de la colonne de purification-réfrigération par contact pour permettre l'injection du distillat de la colonne de fractionnement dans la colonne de purification-réfrigération sans compression dudit distillat.

4. Procédé selon l'une des revendications 1 à 3, caractérisé en ce qu'un appoint de frigories à bas niveau de température, nécessaire au refroidissement à basse température de la charge gazeuse et à sa condensation partielle avant injection dans la colonne de purification-réfrigération, nécessaire au refroidissement du ou des distillats de la colonne de fractionnement, et également nécessaire au système de condensation de cette colonne, est fourni par un système de réfrigération.

5. Procédé selon la revendication 4, caractérisé en ce que le système de réfrigération est constitué par un cycle frigorifique à compression dans lequel un fluide frigorifique comprenant un ou plusieurs corps purs est porté à haute pression dans un compresseur, puis condensé et éventuellement sous-refroidi à cette pression et enfin détendu à la ou les pressions compatibles avec les niveaux de température des systèmes d'échange du procédé pour être vaporisé dans lesdits systèmes.

6. Procédé suivant la revendication 4 ou 5, caractérisé en ce que le système de réfrigération précité est constitué par la détente de la charge gazeuse dans un turbo-expandeur assurant la condensation partielle de ladite charge et produisant un travail mécanique utilisé pour entraîner une machine tournante, tandis qu'un complément de frigories est éventuellement fourni par le cycle frigorifique précité.

7. Installation pour la mise en œuvre du procédé selon l'une des revendications précédentes, et comprenant une colonne (1) de purification-réfrigération par contact associée à une colonne de fractionnement (2, 11), caractérisée par au moins une conduite d'alimentation de distillat (16, 18 ; 38, 40) reliant la tête de la colonne de fractionnement (2, 11) à la tête (1a) de la colonne (1) de purification-réfrigération après passage (17, 39) dans un système d'échange (3) en amont de cette dernière, ainsi que par un conduit (9) reliant le fond de la colonne de purification-réfrigération à la colonne de fractionnement après passage (12) dans ledit système d'échange.

8. Installation selon la revendication 7, caractérisée par au moins une conduite (4) d'arrivée de la charge gazeuse connectée en fond de la colonne (1) de purification-réfrigération et par une conduite (23) d'évacuation du gaz résiduaire reliée à la tête (1a) de ladite colonne, ces deux conduites traversant le système d'échange précité (3).

9. Installation selon la revendication 7 ou 8, caractérisée en ce que la tête de la colonne de fractionnement précitée est munie d'un condenseur (C) et d'un ballon de reflux (15) comportant d'une part un premier conduit (16, 17, 18) d'alimentation en distillat gazeux passant dans le système d'échange (3) avant d'aboutir à la tête (1a) de la colonne (1) de purification-réfrigération, et d'autre part un deuxième conduit (29) de reflux de liquide dans la tête (11a) de la colonne de fractionnement proprement dite, ce deuxième conduit étant éventuellement relié à la tête de la colonne de purification-réfrigération par une conduite (20, 21, 22) traversant ledit système d'échange, pour alimenter dans ladite colonne (1) un distillat liquide sous-refroidi.

10. Installation selon l'une des revendications 7 à 9, caractérisée en ce qu'au système d'échange précité (3) est éventuellement associé un circuit de fluide réfrigérant (28).

11. Installation selon la revendication 7 ou 8, caractérisée en ce que la tête (11a) de la colonne (11) de fractionnement proprement dite est équipée d'un condenseur (41) auquel du froid est fourni par un circuit de fluide réfrigérant associé au système d'échange précité (3).

12. Installation suivant la revendication 7 ou 8, caractérisée en ce que la tête (11a) de la colonne de fractionnement précitée comporte une conduite (71) de distillat vapeur traversant le système d'échange précité (3) et aboutissant à un ballon (15) de reflux du distillat liquide dans ladite colonne, lequel ballon de reflux comporte une conduite (16, 17, 18) d'alimentation en distillat gazeux passant dans ledit système d'échange avant d'aboutir à la tête (1a) de la colonne (1) de purification-réfrigération.

13. Installation suivant la revendication 12, caractérisée en ce que la conduite (4) d'arrivée de la charge gazeuse après passage (5, 62) dans le système d'échange (3) est reliée à un séparateur (66) dont le fond comporte une voie d'écoulement (69) de la fraction liquide dans le fond de la colonne (1) de purification-réfrigération, et dont la tête comporte une voie de passage (78) de la fraction gazeuse aboutissant à l'entrée d'un turbo-expandeur (67) dont la sortie est raccordée (68) au fond de ladite colonne (1).

14. Installation selon la revendication 13, caractérisée en ce que le turbo-expandeur précité (67) entraîne au moins une machine tournante, telle que par exemple un compresseur (75) relié à la conduite (74) d'évacuation du gaz résiduaire.

## Claims

1. Process for the cryogenic fractionation of any gaseous feed into a residual gas containing the most volatile compounds of the feed and into a liquid product containing the heaviest compounds of said feed, consisting in the introduction of said partially condensed charge at the bottom of the purification and refrigeration column by contact, the overhead product of said column consisting of the whole or of a part of said residual gas and the bottom product of said column consisting of a liquid which is fed into a fractionating column in order to obtain at the bottom of this column a liquid product containing the heaviest compounds of the feed, characterized in that the overhead product of the fractionating column is at least partially condensed and fed to the top of the purification and refrigeration column, thus recovering in the bottom liquid of this column the heavy compounds present in the vaporized fraction of the gaseous feed.

2. Process according to claim 1, characterized in that the feed to the top of said purification and refrigeration column consists of a gaseous distillate from the fractionating column, which has been partly or totally condensed in an exchange system, and/or consists of the liquid distillate from said fractionating column, which has been possibly further refrigerated in said exchange system.

3. Process according to claim 1 or 2, characterized in that said fractionating column operates at a higher pressure than the purification and refrigeration column by contact in order to permit the introduction of the distillate from the fractionating column into the purification and refrigeration column without compression of said distillate.

4. Process according to any one of claims 1 to 3, characterized in that an additional refrigeration at a low temperature level, which is necessary for the cooling of the gaseous feed to a low temperature and for its partial condensation before it being introduced into the purification and refrigeration column, which is also necessary for the cooling of the distillate(s) of the fractionating column, and which is also necessary for the condensation system of this column, is provided by a refrigeration system.

5. Process according to claim 4, characterized in that the refrigeration system consists of a refrigeration cycle by compression in which a liquid refrigerant comprising one or more pure substances is compressed up to a high pressure in a compressor, then is condensed and is possibly further cooled under this pressure, and is finally expanded to the pressure(s) which is (are) compatible with the temperature levels of the exchange systems of the process, in order to be vaporized in said systems.

6. Process according to claim 4 or 5, characterized in that said refrigeration system consists of the expansion of the gaseous feed in a turbo-expander performing the partial condensation of said feed and producing a mechanical work which is used to drive a rotating machine, whereas and additional refrigeration is possibly produced by said refrigeration cycle.

7. Installation for performing the process according to any preceding claim, comprising a purification and refrigeration column by contact (1) combined with a fractionating column (2, 11), characterized in that at least one feeding pipe (16, 18 ; 38, 40) for the distillate connects the top of the fractionating column (2, 11) with the top (1a) of the purification and refrigeration column (1) after passing (17, 39) through an exchange system (3) upstream of the column (1) and in that a pipe (9) connects the bottom of the purification and refrigeration column with the fractionating column after passing (12) through said exchange system.

8. Installation according to claim 7, characterized in that at least one inlet pipe (4) for the gaseous feed is connected with the bottom of the purification and refrigeration column (1) and in that an outlet pipe (23) for the residual gas is connected with the top (1a) of said column, both pipes passing through said exchange system (3).

9. Installation according to claim 7 or 8, characterized in that the top of said fractionating column is equipped with a condenser (C) and with a reflux drum (15) which comprises, on the one hand, a first feeding pipe (16, 17, 18) for the gaseous distillate passing through the exchange system (3) and then arriving at the top (1a) of the purification and refrigeration column (1) and, on the other hand, a second pipe (29) for the reflux of liquid to the top (11a) of the fractionating column itself, whereas this second pipe may be connected with the top of the purification and refrigeration column through a pipe (20, 21, 22) passing through said exchange system, in order to feed into said column (1) a liquid distillate which has undergone an additional refrigeration.

10. Installation according to any one of claims 7 to 9, characterized in that a liquid refrigerant circuit (28) may be associated with said exchange system (3).

11. Installation according to claim 7 or 8, characterized in that the top (11a) of the fractionating column (11) itself is equipped with a condenser (41) to which a refrigeration is brought by a liquid refrigerant circuit associated with said exchange system (3).

12. Installation according to claim 7 or 8, characterized in that the top (11a) of said fractionating column includes a pipe (71) for the vapour distillate, which passes through said exchange system (3) and which arrives at a reflux drum (15) for refluxing the liquid distillate into said column, said reflux drum including a feed pipe (16, 17, 18) for the gaseous distillate, which passes through said exchange system and then arrives at the top (1a) of the purification and refrigeration column (1).

13. Installation according to claim 12, characterized in that the inlet pipe (4) for the gaseous feed, after passing (5, 62) through the exchange system (3) is connected with a separator (66), the bottom of which includes a pipe (69) for the flowing of the liquid fraction into the bottom of the purification and refrigeration column (1), and the top of which includes a circulation pipe (78) for the gaseous fraction, which arrives at the inlet of a turbo-expander (67), the outlet of which is connected (68) to the bottom of said column (1).

14. Installation according to claim 13, characterized in that said turbo-expander (67) drives at least one rotating machine, such as for example a compressor (75), which is connected to the pipe (74) for the withdrawal of the residual gas.

**Patentansprüche**

1. Verfahren zur kryogenischen Fraktionnierung irgendwelcher gasförmigen Masse zu einem, die flüchtigsten Verbindungen der Masse enthaltenden Restgas und zu einem, die schwersten Verbindungen dieser Masse enthaltenden flüssigen Produkt, bestehend aus der Einspeisung der besagten, teilweise kondensierten Masse in den Unterteil einer Kontaktkolonne zur Reinigung und Kühlung, wobei diese Kolonne das besagte Restgas vollständig oder teilweise als Kopfprodukt erzeugt und eine Flüssigkeit als Sumpfprodukt erzeugt, welches in eine Fraktionnierungskolonne eingeleitet wird, damit im Unterteil dieser Kolonne ein flüssiges, die schwersten Produkte der Masse enthaltendes Produkt, erhalten wird, dadurch gekennzeichnet, dass das Kopfdestillat der Fraktionnierungskolonne wenigstens teilweise kondensiert und als Kopfzufuhr in die Kolonne zur Reinigung und Kühlung eingespeist wird, zur Zurückgewinnung der sich in der verdampften Fraktion der gasförmigen Masse befindenden schweren Verbindungen in der sich im Sumpf dieser Kolonne befindenden Flüssigkeit.

2. Verfahren nach Anspruch 1, dadurch gekennzeichnet, dass die Kopfzufuhr der besagten Kolonne zur Reinigung und Kühlung aus dem dampfförmigen Destillat der Fraktionnierungskolonne, welches vollständig oder teilweise in einem Austauschsystem kondensiert ist, und/oder aus dem flüssigen Destillat der besagten Fraktionnierungskolonne, welches im besagten Austauschsystem gegebenenfalls weitergekühlt ist, besteht.

3. Verfahren nach Anspruch 1 oder 2, dadurch gekennzeichnet, dass die besagte Fraktionnierungskolonne bei einem, gegenüber dem in der Kontaktkolonne zur Reinigung und Kühlung herschenden Druck, höheren Druck arbeitet, damit die Einspeisung des aus der Fraktionnierungskolonne kommenden Destillats in die Kolonne zur Reinigung und Kühlung ohne Verdichtung des besagten Destillats gewehrleistet ist.

4. Verfahren nach einem der Ansprüche 1 bis 3, dadurch gekennzeichnet, dass eine zustätzliche Kühlung mit niedrigem Temperaturniveau, die notwendig ist sowohl für die Tieftemperaturkühlung der gasförmigen Masse und für die Teilkondensation derselben bevor die Masse in die Kolonne zur Reinigung und Kühlung eingeleitet ist, als auch für die Kühlung des Destillats (Destillate) der Fraktionnierungskolonne, und zuletzt auch für das Kondensationssystem dieser Kolonne, aus einem Kühlungssystem geliefert ist.

5. Verfahren nach Anspruch 4, dadurch gekennzeichnet, dass das Kühlungssystem aus einem Verdichtungskältekreislauf besteht, in welchem ein Kältemittel bestehend aus einem oder aus mehreren reinen Stoffen, in einem Verdichter zu einem hohen Druck verdichtet, dann kondensiert und gegebennenfalls unter demselben Druck weitergekühlt und zuletzt zu dem (zu der) mit den Temperaturniveaus der Austauschsystemen des Verfahrens verträglichen Druck (Drücken) entspannt wird, zur Verdampfung desselben in den besagten Systemen.

6. Verfahren nach Anspruch 4 oder 5, dadurch gekennzeichnet, dass das besagte Kühlungssystem aus der Entspannung der gasförmigen Masse in einem Turbo-Expander besteht, wobei der Turbo-Expander die Teilkondensation der besagten Masse gewährleistet und eine mechanische Arbeit erzeugt, die zum Antrieb einer rotierenden Maschine benutzt wird, während eine zusätzliche Kühlung von besagten Kältekreislauf gegebenenfalls geliefert wird.

7. Vorrichtung zur Durchführung des Verfahrens nach einem der vorstehenden Ansprüche, bestehend aus einer Kontaktkolonne (1) zur Reinigung und zur Kühlung in Verbindung mit einer Fraktionnierungskolonne (2, 11), gekennzeichnet durch wenigstens eine Zufuhrleitung (16, 18 ; 38, 40) für das Destillat, die den Kopf der Fraktionnierungskolonne (2, 11) mit dem Kopf (1a) der Kolonne (1) zur Reinigung und Kühlung verbindet, nachdem sie ein sich oberhalb der Kolonne (1) befindendes Austauschsystem (3) durchquert hat, sowie duch eine Leitung (9) die den Unterteil der Kolonne zur Reinigung und Kühlung mit der Fraktionnierungskolonne verbindet, nachdem die Leitung (9) das besagte Austauschsystem durchquert hat (12).

8. Vorrichtung nach Anspruch 7, gekennzeichnet durch wenigstens eine Zufuhrleitung (4) für die gasförmige Masse, die in den Unterteil der Kolonne (1) zur Reinigung und Kühlung gelangt, und durch eine Abzugsleitung (23) für das Restgas, die mit dem Kopf (1a) der besagten Kolonne in Verbindung steht, wobei beide Leitungen das besagte Austauschsystem (3) durchqueren.

9. Vorrichtung nach Anspruch 7 oder 8, dadurch gekennzeichnet, dass der Kopf der besagten Fraktionnierungskolonne mit einem Kühler (C) und einem Rückflussbehälter (15) ausgestattet ist, wobei der Rückflussbehälter einerseits eine erste Zufuhrleitung (16, 17, 18) für das das Austauschsystem 3 überquerende und anschliessend in den Kopf (1a) der Kolonne (1) zur Reinigung und Kühlung gelangende gasförmige Destillat aufweist, und andererseits eine zweite Leitung (29) für den Flüssigkeitsrückfluss in den Kopf (11a) der eigentlichen Fraktionnierungskolonne aufweist, wobei diese zweite Leitung mit dem Kopf der Kolonne zur Reinigung und Kühlung durch eine, das besagte Austauschsystem überquerende Leitung (20, 21, 22) gegebenenfalls verbunden ist, zur Einspeisung eines flüssigen, weitergekühlten Destillats in die besagte Kolonne (1).

10. Vorrichtung nach einem der Ansprüche 7 bis 9, dadurch gekennzeichnet, dass ein Kühlkreis (28) mit dem besagten Austauschsystem (3) gegebenenfalls verbunden ist.

11. Vorrichtung nach Anspruch 7 oder 8, dadurch gekennzeichnet, dass der Kopf (11a) der eigentlichen Fraktionnierungskolonne (11) mit einem Kühler (41) ausgestattet ist, zu dem Kälte von einem mit dem besagten Austauschsystem (3) verbundenen Kühlkreis geliefert ist.

12. Vorrichtung nach Anspruch 7 oder 8, dadurch gekennzeichnet, dass der Kopf (11a) der besagten Fraktionnierungskolonne eine, das besagte Austauschsystem (3) überquerende und zu einem Behälter (15) zum Rückfluss des flüssigen Destillats in die besagte Kolonne ankommende Leitung (71) für das gasförmige Distillat aufweist, wobei der besagte Ruckflussbehälter eine Zufuhrleitung (16, 17, 18) für das gasförmige Destillat aufweist, die das besagte Austauschsystem überquert und anschliessend in den Kopf (1a) der Kolonne (1) zur Reinigung und Kühlung gelangt.

13. Vorrichtung nach Anspruch 12, dadurch gekennzeichnet, dass die Zufuhrleitung (4) für die gasförmige Masse, nachdem sie das Austauschsystem (3) überquert hat (5, 62), mit einem Abscheider (66) verbunden ist, dessen Unterteil eine Leitung (69) zur Strömung der flüssigen Fraktion in den Unterteil der Kolonne (1) zur Reinigung und Kühlung aufweist, und dessen Kopf eine Leitung (78) zum Durchlauf der gasförmigen Fraktion aufweist, die in den Einlass eines Turbo-Expanders (67) gelangt, dessen Auslass mit dem Unterteil der besagten Kolonne (1) verbunden ist (68).

14. Vorrichtung nach Anspruch 13, dadurch gekennzeichnet, dass der besagte Turbo-Expander (67) wenigstens eine rotierende Maschine, wie zum Beispiel einen mit der Ausfuhrleitung (74) des Restgases verbundenen Verdichter (75), antreibt.

Fig. 1

Fig. 2

0 178 207

Fig. 3

0 178 207